# EUROPEAN PATENT SPECIFICATION

(11) **EP 3 130 594 B1**
(45) Date of publication and mention of the grant of the patent: **11.11.2020**
(21) Application number: 16183580.6
(22) Date of filing: 10.08.2016
(51) Int. Cl.: C07F 9/40, A61K 31/56, A61P 35/00, C07J 63/00, A61K 31/662

(54) **PHOSPHONATES OF ACETYLENIC BETULIN DERIVATIVES WITH ANTICANCER ACTIVITY, METHOD FOR THEIR PRODUCTION AND THEIR APPLICATION**
PHOSPHONATE VON ACETYLENBETULINDERIVATEN MIT ANTIKREBSAKTIVITÄT, VERFAHREN ZU DEREN HERSTELLUNG UND DEREN ANWENDUNG
PHOSPHONATES DE DÉRIVÉS ACÉTYLÉNIQUES DE BÉTULINE PRÉSENTANT UNE ACTIVITÉ ANTICANCÉREUSE, PROCÉDÉ POUR LEUR PRODUCTION ET LEUR APPLICATION

(30) Priority: 13.08.2015 PL 41351615
(43) Date of publication of application: 15.02.2017
(73) Proprietor: Slaski Uniwersytet Medyczny w Katowicach, 40-055 Katowice (PL)
(72) Inventor: Boryczka, Stanislaw, 41-209 Sosnowiec (PL); Chrobak, Elwira, 40-877 Katowice (PL); Latocha, Malgorzata, 40-588 Katowice (PL); Kadela, Monika, 40-018 Katowice (PL); Bebenek, Ewa, 41-300 Dabrowa Górnicza (PL)
(74) Representative: Wroblewski, Michal

(56) References cited:
- JP-A- 2011 225 538
- ALOIS VYSTRCIL ET AL: "Isomeric oximes of 30-norlupan-20-one and its derivatives", COLLECTION SYMPOSIUM SERIES (XIIITH SYMPOSIUM ON CHEMISTRY OF NUCLEIC ACID COMPONENTS SPINDLERUV MLYN, CZECH REPUBLIC; SEPTEMBER 03 -09, 2005), vol. 51, no. 3, 1986, pages 581-592, XP55330336, XX ISSN: 0010-0765, DOI: 10.1135/cccc19860581 ISBN: 978-80-86241-25-8 & DATABASE REAXYS [Online] Elsevier; 1986, Vystrcil, Alois: XP002765367, Database accession no. 5203916

## Description

The invention relates to phosphonates of acetylenic betulin derivatives with anticancer activity, method for their production and application.

Betulin [lup-20(29)-ene-3β,28-diol] is a pentacyclic triterpenoid of lupane type, commonly occurring naturally. Large amounts of betulin are found in the external layer of bark of white birch species (25-30% content), therefore the bark is a readily available raw material (being a paper factory by-product) for preparation of betulin by an extraction process, not requiring high capital expenditures, even on an industrial scale (US Pat 6392070B1). Betulin and its easily obtainable derivatives exhibit a broad range of biological activity, such as: anticancer, antibacterial, antiviral, anti-inflammatory, hepatoprotective, anticholelithiasis, and others, occurring at very low concentrations without toxicity both *in vitro,* and *in vivo* (T.G. Tolstikova, I.V. Sorokina, G.A. Tolstikov, O.B. Flekhter, Russ. J. Bioorg. Chem., 2006, 32, 37-49; T.G. Tolstikova, I.V. Sorokina, G.A. Tolstikov, O.B. Flekhter, Russ. J. Bioorg. Chem., 2006, 32, 261-276; S. Alakurtti, T. Makela, S. Koskimies, J.Yli-Kauhaluoma, Eur. J. Pharm. Sci., 2006, 29, 1-13). Due to these features, betulin (Formula 1), known for more than 200 years, containing two hydroxy groups at C3 and C28, and an isopropenyl group at C19, is a very good starting material for preparation of new derivatives with better pharmacological properties, and such works are being undertaken in numerous scientific centers globally, which is proved by the number of published papers.

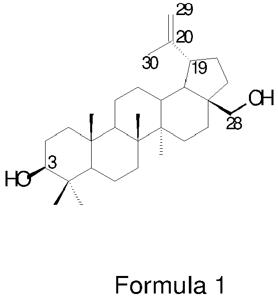

Betulin may be oxidised easily to betulonic acid, from which, after a selective reduction of the ketone group at C3 atom, betulinic acid ((3β)-3-hydroxy-lup-20(29)- en-28-oic acid) is obtained, having a high anticancer activity (ED₅₀ = 0.5-4.0 µg/ml), particularly towards some neoplasms resistant to chemotherapy, such as melanomas and gliomas. The mechanism of betulinic acid action consists in inducing apoptosis only in diseased cells. Thanks to this fact, a part of unfavourable side effects connected with anticancer chemotherapy may be excluded (R. Mukherjee, V. Kumar, S. K.Srivastava, S.K. Agarwal, A.C. Burman, Anti-Cancer Agents Med. Chem., 2006, 6, 271-279, EP1266658A2, US6048847A).

Known synthetic modifications of betulin, betulinic acid and betulonic acid include obtaining of such derivatives as: monophthalate, diphthalate, succinate, diacetate, dipropionate, dibutyrate, mono- and dinicotinate, acetylsalicylate, cinnamate, as well as amide, carbohydrate, amine acid, carbamate and N-acylheterocyclic derivatives, described, among others, in the following publications: O.B. Flekhter, L.T. Karachurina, L.R. Nigmatullina, T.A. Sapozhnikova, L.A. Baltina, F.S. Zarudii, F.Z. Galin, L.V. Spirikhin, G.A.Tolstikov, O.A. Plyasunova, A.G. Pokrovskii, Russ. J. Bioorg. Chem., 2002, 28, 494-500; O.B. Flekhter, N.I. Medvedeva, L.T. Karachurina, L.A. Baltina, F.Z. Galin, F.S. Zarudii, G.A. Tolstikov, Pharm. Chem. J., 2005, 39, 401-404; S. Alakurtti, T. Makela, S. Koskimies, J. Yli-Kauhaluoma, Eur. J. Pharm.Sci., 2006, 29, 1-13; D.S.H.L. Kim, J.M. Pezzuto, E. Pisha, Bioorg. Med. Chem. Lett., 1998, 8, 1707-1712; F. Soler, C. Poujade, M. Evers, J-C. Carry, Y. Henin, A. Bousseau, T. Huet, R. Pauwels, E. De Clercq, J-F. Mayaux, J-B. Le Pecq, N. Dereu, J. Med. Chem., 1996, 39, 1-69- 1083; Y. Kashiwada, J. Chiyo, Y. Ikeshiro, T. Nagao, H. Okabe, L.M. Cosentino, K. Fowke, K.H. Lee, Bioorg. Med. Chem. Lett., 2001, 11, 183-185; Y. Kashiwada, M. Sekiya, Y. Ikeshiro, T. Fujioka, N.R. Kilgore, C.T. Wild, G.P. Allaway, K-H. Lee, Bioorg. Med. Chem. Lett., 2004, 14, 5851-585; M. Kvasnica, J. Sarek, E. Klinotova, P. Dzubak, M. Hajduch, Bioorg. Med. Chem. Lett., 2005, 13, 3447- 3454: R.H. Cichewicz, S.A. Kouzi, Med. Chem. Rev., 2003, 24, 90-114; I. Baglin, A-C. Mitaine-Offer, M. Nour, K. Tan, C. Cave, M-A. Lacaille-Dubois, Mini Rev. Med. Chem., 2003, 3, 525-539; G.A. Tolstikov, O.B. Flekhter, E.E. Shultz, L.A. Baltina, A.G. Tolstikov, Chem. Sustain. Develop., 2005, 13, 1-29, H. Kommera, G.N. Kaluderović, S. Dittrich, J. Kalbitz, B. Drager, T. Mueller, R. Paschke, Bioorg. Med. Chem. Lett., 2010, 20, 3409-3412, R.C. Santos, J.A.R. Salvador, S. Marfn, M. Cascante, J.N. Moreira, T.C.P. Dinis, Bioorg. Med. Chem., 2010, 18, 4385- 4396, as wel! as disclosed in the following patent descriptions: US5679828, US5468888, Jap.01143 832, US617211O, WO 2004/028455A2, US6048841, WO2006/105356A2, US6407270B1, WO 2006/133314A2, WO2007/141392A2, WO2008/070347 A2, US2003/0181429A1, US6403816B1, US6228850B1, WO2006/085334A2.

JP2011/225538 discloses Betulin derivatives featuring a propen-2-yl group attached to the C17 atom of the D-ring; Alois Vystrcil et al disclose in XIIIth Symposium on Chemistry of Nucleic Acid Components, Czech Republic 2005, Vo 51, 1986, pages 581-592 steroids to which a phosphate group is attached in C17-position.

In spite of the fact that betulin and its synthetically modified derivatives have interesting pharmacological properties, development of effective anticancer drugs from this group of compounds and their introduction onto the pharmaceutic market have not succeeded hitherto. The most promising cytostatics include betulinic acid, currently being in the phase of advanced clinical tests (G.A. Tolstikov, O.B. Flekhter, E.E. Shultz, L.A. Baltina, A.G. Tolstikov, Chem. Sustain. Devefop., 2005, 13,1-29).

Carbon-carbon triple bond is considered one of the most important functional groups in organie chemistry and medical chemistry. It results from the fact that a pharmacophore of such a type easily undergoes to numerous transformations; it occurs in natural compounds; as well as significantly modifies the biological properties of a compound. Mono- or disubstituted derivatives obtained in reactions of betulin with propiolic acid or propargyl chloroformate with a high cytotoxic activity against CCRF/CEM cells of human leukemia and P388 cells of murine leukemia, while compared to cisplatin, deserve a particular attention (S. Boryczka, E. 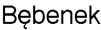 , J. Wietrzyk, K. Kempińska, M. 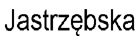 , J. Kusz, and M. Nowak, *Mo*/*ecu*/*es,* 2013, 18, 4526-4543). In the paper by Y. Bi, J. Xu, X. Wu, W. Ye, S. Yuan, L. Zhang (Bioorg. Med. Chem. Lett., 2007, 17, 1475- 1478) propargyl and 2-butynyl esters of 23-hydroxybetulinic acid with a cytotoxic activity close to that of 23-hydroxybetulinic acid were described.

R. Csuk *et al.* described synthesis of 3-propargylamine derivatives of betulinic acid, exhibiting a higher cytotoxic activity against the lung cancer A549 cell line that the starting betulinic acid (R. Csuk, C. Nitsche, R. Sczepek, S. Schwarz, and B. Siewert, Arch. Pharm. Chem. Life Sci,. 2013, 346, 232-246). Acetylenic derivatives of betulin were transformed into new triazolic derivatives, which also exhibit a cytotoxic activity (R. Csuk, A. Barthel, R. Kluge, D. Strohlet, Bioorg. Med. Chem., 2010, 18, 7252-7259, R. Csuk, A. Barthel, R. Sczepek, B. Siewert, S. Schwarz, Arch. Pharm. Chem. Life Sci. 2011, 1, 37-49).

Another known literature description: S.F. Vasilevsky, A.I. Govdi, E.E. Shults, M.M. Shakirov, I.V. Sorokina, T.G. Tolstikova, D.S. Baev, G.A. Tolstikov, I.V. Alabugin, Bioorg. Med. Chem., 2009, 17, 5164-5169; S.F. Vasilevsky, A.I. Govdi, E.E. Shults, M.M. Shakirov, I.V. Alabugin, G.A. Tolstikov, Doklady Chem., 2009, 424, 39-42 pertain to synthesis of arylalkynyl amides of betulonic acid with hepatoprotective and anti-inflammatory properties.

An antiviral activity comparable with that of AZT was described for bevirimat propargyl ester (I.O. Bori, H-Y. Hung, K. Qian, C-H. Chen, S. L. Morris-Natschke, K-H. Lee, Tetrahedron Lett, 2012, 53, 1987-1989).

The U.S. patent description US2003/0181429A1 discloses derivatives of 3-alkynylaminobetulinic acid and 3-(alkynyloxyimino)-betulinic acid as components of mixtures with activities close to that of betulinic acid, which may find application in prevention and inhibition of growth of cancer cells.

Patent descriptions WO2006/105356A2, US640727081, US6867314B2, and US 2003/0073858A1 pertain to synthesis of mono- and diesters of betulinic acid, and betulinic aldehyde containing also alkynyl groups (C2-C10). As acylating agents, difficult to obtain chlorides, anhydrides and acetylenic acids are used. The reactions are carried out in elevated temperatures, which may affect the stability of the acetylenie substituents in the final products unfavourably.

Phosphonates are compounds occurring commonly in living organisms, performing various functions; among others, they are a group of compounds with an antibiotic effect. Phosphomycin isolated from various *Streptomyces* strains is an important antibiotic with a vast application range. It exerts a strong effect against Gram-negative bacteria and Gram-positive bacteria (N. Roussos, D.E. Karageorgopoulos, G. Samonis, M.F. Falagas, Int. J. Antimicrob. Agents, 2009, 34, 506-515). Fosmidomycin is an antibiotic exhibiting antimalarial activity (B. Lell, R. Ruangweerayut, J. Wiesner, M. Anoumou Missinou, A. Schindler, T. Baranek, M. Hintz, D. Hutchinson, H. Jomaa, and P.G. Kremsner, Antimicrob. Agents Chemother., 2003, 47, 735-738).

In the result of the search for new substances exhibiting an antibacterial action, phosphonic chromene derivatives were obtained and described (M. Rajasekhar, K.U.M. Rao, C.S. Sundar, N.B Reddy, S.K Nayak, C.S Reddy, Chem. Pharm. Bull. 2012; 60, 854-858), as well as diphenyl esters of α-hydroxy alkyl- and arylphosphonates (A.M.F. Phillips, M.T. Barros, M. Pacheco, R. Dias, Bioorg. Med. Chem. Lett., 2014, 24, 49-53).

Phosphonates with antiviral action, such as: cidofovir, foscarnet, tenofovir or adefovir were introduced to therapeutics (K.K. Biron, Antiviral. Research 2006, 71, 154-163; M. Dracinsky, M. Krecmerova, A. Holy, Bioorg. Med. Chem., 2008, 16, 6778-6782). Cidofovir exhibits an activity against a broad range of DNA viruses, it is active against HSV-1, HSV-2, Epstein-Barr virus, strongly inhibits growth of human herpes viruses (HHV-6, HHV-7, HHV-8), TK-HSV, TK-Varicella-Zoster. It is also active against adenoviruses, papillomaviruses, poxviruses, and parapoxviruses (P. Broganelli, A. Chiaretta, B. Fragnelli, M.G. Bernengo, Dermatol. Ther., 2012, 25, 468-471.)
The U.S. patent description (US2013/0225532A1) discloses phosphonic compounds active against wild strains of human influenza virus and oseltamivir - resistant avian influenza virus H1N1, H5N1, and H3N2.

For many years, bisphosphonate derivatives such as: alendronate, etindronate, ibandronate, clodronate, pamidronate, risedronate, tiludronate and zoledronate have been used in osteoporosis therapy (H. Fleisch., Endocr. Rev., 19 1998 80-100).
An important group of phosphonic derivatives is constituted by hydroxyphosphonates, among which human renin inhibitors exhibiting the effect of reduction of arterial blood pressure have been described (J.F. Dellaria, Jr., R.G. Maki, H.H. Stein, J. Cohen, D. Whittern, K. Marsh, D.J. Hoffman, J.J. Plattner, T.J. Perun, J. Med. Chem., 1990, 33, 534-542; O.V. Patel, K. Rielly-Gauvin, D.E. Ryono, C.A. Free, W.L. Rogers, S.A. Smith, J.M. DeForrest, E.W. Oehl, E.W. Petrillo Jr., J. Med. Chem., 1995, 38, 4557-4569).

Over recent years, studies on the anticancer activity of α-aminophosphonic derivatives, as well as phosphonates already being used as drugs, e.g. foscarnet and bisphosphonates, were carried out too (K.A. Mungara, Y-K. Park, K.D. Lee. Chem. Pharm. Bull., 2012; 60, 1531-1537; C.B. Reddy, K.S. Kumar, MA Kumar, M.V.N. Reddy, B.S. Krishna, M. Naveen, M.K. Arunasree, C.N. Reddy, C.N. Raju, C.D. Reddy, Eur J. Med. Chem. 2012; 47, 553-559; K. Rose, Biomed. Pharmacother. 2013, 67:53-57, E.D. Carlo, P. Bocca, L. Emionite, M. Cilli, G. Cipollone, F. Morandi, L. Raffaghello, V. Pistoia, I. Prigione, Am. Soc. Gen. Cell Ther. 2013; 21:1034-1043).

Japanese Patent Application JP 2011 225538 by Takatsu Kiyoshi, Matsunaga Takayuki, Ogasawara Masaru, concerns research on the possibility of using betulin, betulinic acid, lupeol and betulin diacetate in mixtures that can be used in cancer immunotherapy.

Up to now, in spite of these valuable properties of phosphonates, little attention was paid to chemical modifications of betulin and its derivatives using phosphonic pharmacophoric groups. As yet, no betulin derivatives containing phosphonic moieties in their structure were described in the chemical literature. Introduction of a phosphonic group, as a group bioisosteric to phosphate group, allows for avoiding the first phosphorylation step and simultaneously ensure stability of phosphonates under conditions of enzymatic hydrolysis. Free phosphonic acids, because of the high negative charge of the phosphoryl moiety, penetrate biological membranes more poorly in comparison to the corresponding phosphonic esters. Thus, the synthesis of esters of phosphonic acids becomes more and more important in designing new phosphonates.

The goal of the present invention is to prepare new compounds with anticancer activity, having structure based on the structure of betulin molecule and their derivatives with phosphonic groups as well as, additionally, with acetylenic pharmacophoric moieties. The goal and advantage of the method according to the invention consists in using commercially available chemicals, realisation of the process under mild conditions with high yields, and most of all, using betulin as the main substrate, which may be readily obtained for instance from birch bark, being a waste material from paper production. In the result of the studies carried out, it turned out unexpectedly that the introduction of a phosphonic group at position 29 or 30 of betulin, followed by the introduction of one or two the same or different acetylenic substituents at positions 3 and/or 28 of betulin and its derivatives, allows for obtaining compound with a high cytotoxic activity *in vitro* against human cells of cancer lines, while compared to the betulin compounds described hitherto.

The compounds according to the invention have a chemical structure described by 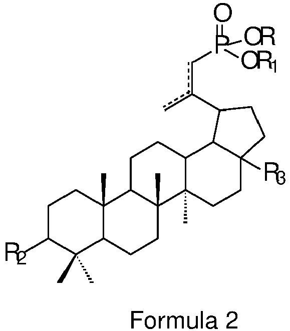 wherein a phosphonic group is bounded with C29 or C30 carbon atom of the isopropenyl group, wherein the dashed lines between C20, and C29 and C30 indicate that the bond between them may be a double or single bond, and the individual substituents are as follows:
R = R₁ is alkyl group (C1-C4)
R₂ is hydroxy, propynoyloxy, cyclopropylpropynoyloxy, 2-butynoyloxy, propargyloxycarbonyloxy group,
R₃ is hydroxymethyl, propynoyloxymethyl, cyclopropylpropynoyloxymethyl, 2-butynoyloxymethyl, propargyloxycarbonyloxymethyl group

In a method for preparation of the compounds with Formula 2 according to the invention, the compound with Formula 3 obtained from betulin isolated from birch bark is used as a substrate for the synthesis of phosphonic betulin derivatives.

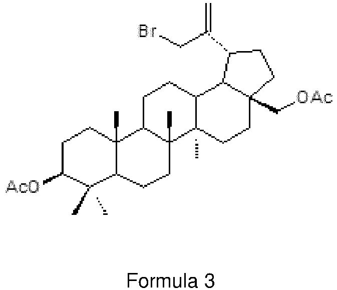

The method for preparation of phosphonates of acetylenic betulin derivatives with Formula 2 according to the invention consists in that the compound with Formula 3 is reacted with a trialkyl phosphite at a temperature of 100°C-165°C, giving a product with Formula 4 wherein R = R₁ is alkyl group (C1-C4)

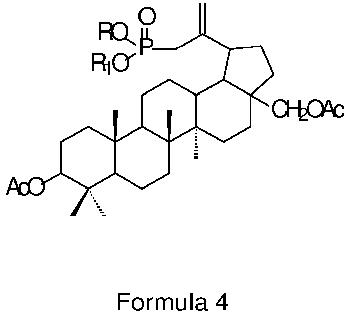

The obtained compound is subjected to hydrolysis in alkaline medium in the presence of KOH in ethanolic solution, at boiling temperature, giving a mixture of allyl-vinyl rearrangement products with Formula 2B (trans) and 2C (cis), wherein R = R₁ = C1-C4 alkyl, or in the presence of NaOH in CH₃OH/THF/H₂O solution at room temperature, giving compound with Formula 2A, wherein R = R₁ is alkyl group (C1-C4).

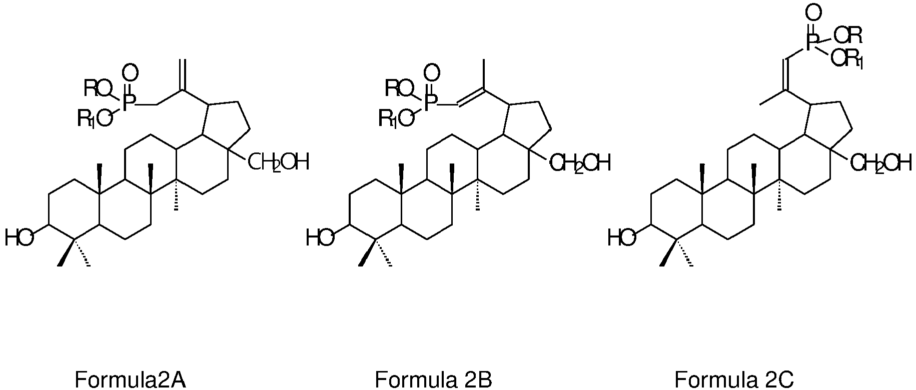

A method for preparation of acetylenic betulin derivatives with Formula 2 according to the invention, utilises easily available substrates, gives good reaction yields i.e. 61-90% (in relation to monoesters) and 7-20% (in relation to diesters), moreover the isolation method is simple. Structures of the compounds were confirmed based on ¹H NMR, ¹³C NMR, ³¹P NMR, and IR spectroscopies.

The compounds according to the invention exhibit cytotoxic activity against cells from various cancer lines: brain glioma (SNB19), breast carcinoma (T47D), and melanoma (C32). For the sake of the obtained activity, these compounds preferably may be used separately as compounds inhibiting proliferation of cancer cells.

Moreover, the compounds included in the invention may be precursors in preparation of further effective cytostatics via functionalization of acetylenic substituents, being their additional advantage.

The method according to the invention is described in more detail in the embodiments which do not limit the scope of the invention in any way.

### Example 1. Preparation of phosphonates 4 (R = R₁ = Me, Et).

30-bromo-3,28-diacetylbetulin 3 (0.606 g, 1 mmol) and 20 mmol of trimethyl phosphite or triethyl phosphite is placed in a round-bottomed flask protected from humidity, under argon atmosphere. The reaction is carried out at boiling temperature of the mixture for 6 hours. Then, the excess of the phosphite is distilled off, and the remainder is concentrated till dry on a vacuum evaporator. The obtained product is washed with hexane, then purified by column chromatography (SiO₂, chloroform/acetone, 7:3 *vlv).* Product 4 is obtained (R = R₁= Me, Et).
a) 3, 28 - Diacetyl - 30 - dimethoxyphosphorylbetulin 4 (R = R₁ = Me)
   Yield: 70%. Mp. 90-94°C.
   TLC (chloroform/ethyl acetate, 7:3, *vlv);* R_{f}= 0.19.
   Spectroscopic data of the obtained product are as follows:
   ¹H-NMR (CDCl₃) δ (ppm): 0.80 (m, 1H, H-5), 0.85 (s, 3H, CH₃), 0.86 (s, 6H, 2xCH₃), 0.98 (s, 3H, CH₃), 1.05 (s, 3H, CH₃), 0.81-2.10 (m, 23H, CH, CH₂), 2.07 (s, 3H, COCH₃), 2.09 (s, 3H, COCH₃), 2.44 (m, 1H, H-19), 2.62 (m, 2H, H-30), 3.76 (s, 3H, POCH₃), 3.78 (s, 3H, POCH₃), 3.82 (d, *J* = 10.8 Hz, 1H, H-28), 4.28 (d, *J* = 10.8 Hz, 1H, H-28), 4.49 (m, 1H, H-3), 5.03 (br. s., 1H, H-29), 5.07 (br. s., 1H, H-29).
   ¹³C-NMR (CDCl₃) δ (ppm): 14.2, 14.7; 16.0; 16.2; 16.5; 18.2; 20.9; 21.0; 21.1, 21.3; 23.7; 26.4, 27.0; 27.9; 29.8; 34.1; 34.3; 37.0; 37.4; 37.8; 38.4; 40.9; 42.7; 46.3; 50.0; 50.2; 52.6, 52.7, 55.4; 60.4; 62.7; 80.9; 113.2; 144.4; 171.1; 171.6.
   ³¹P-NMR (CDCl₃) δ (ppm): 30.26.
   IR (KBr, cm⁻¹) v: 1734 (C=O), 1246 (P=O), 1031(P-O-R), 800 (P-C).
b) 3, 28 - Diacetyl - 30 - diethoxyphosphorylbetulin 4 (R = R₁ = Et)
   Yield: 76%. Mp. 152-155°C.
   TLC (chloroform/ethyl acetate, 7:3, v/v); R_{f} = 0.38.
   Spectroscopic data of the obtained product are as follows:
   ¹H-NMR (CDCl₃) δ (ppm): 0.80 (m, 1H, H-5), 0.85 (s, 3H, CH₃), 0.86 (s, 6H, 2xCH₃), 0.99 (s, 3H, CH₃), 1.05 (s, 3H, CH₃), 1.34 (m, 6H, 2xOCH₂CH₃), 0.81-2.10 (m, 23H, CH, CH₂), 2.06 (s, 3H, COCH₃), 2.09 (s, 3H, COCH₃), 2.46 (m, 1H, H-19), 2.60 (m, 2H, H-30), 3.83 (d, *J* = 10.8 Hz, 1H, H-28), 4.13 (m, 4H, 2x OCH₂CH₃), 4.28 (d, *J* = 10.8 Hz, 1H, H-28), 4.49 (m, 1H, H-3), 5.01 (br. s, 1H, H-29), 5.07 (br. s, 1H, H-29).
   ¹³C-NMR (CDCl₃) δ (ppm): 14.1; 14.8; 16.0; 16.1; 16.4; 16.5; 18.2; 20.9; 21.0; 21.3; 23.7; 26.4; 27.0; 27.9; 29.8; 31.6; 34.1; 34.2; 37.0; 37.4; 37.8; 38.4; 40.9; 42.7; 46.3; 49.9; 50.2; 55.4; 61.8; 61.9; 62.7; 80.9; 112.8; 144.6; 171.0; 171.5.
   ³¹P-NMR (CDCl₃) δ (ppm): 27.73.
   IR (KBr, cm⁻¹) v: 1741 (C=O), 1248 (P=O), 961 (P-O-R), 784 (P-C).

### Example 2. Preparation of trans-2B and cis-2C phosphonates (R = R₁ = Et)

Compound 4 (R = R₁ = Et) (0.663 g, 1 mmol), 10 ml of ethanol and 0.308 g of potassium hydroxide are placed in a flask and the whole mixture is heated at boiling temperature for 2 hours. Then, the post-reaction mixture is concentrated till dry on a vacuum evaporator. The raw product is purified by column chromatography (SiO₂, methylene chloride/ethanol, 15:1 v/v) giving pure isomers *trans-2B* and *cis-2C* (R = R₁ = Et).
a) E-29-Diethoxyphosphorylbetulin *(trans-2* B) (R = R₁ = Et)
   Yield: 64%. Mp. 120-123°C.
   TLC (methylene chloride/ethanol, 15:1, v/v); R_{f} = 0.31. Spectroscopic data of the obtained product are as follows:
   ¹H-NMR (CDCl₃) δ (ppm): 0.70 (m, 1H, H-5), 0.78 (s, 3H, CH₃), 0.84 (s, 3H, CH₃), 0.99 (s, 3H, CH₃), 0.994 (s, 3H, CH₃), 1.07 (s, 3H, CH₃), 1.33 (m, 6H, 2xOCH₂CH₃), 0.88-2.08 (m, 25H, CH, CH₂), 2.07 (d, *J* = 3Hz, 3H, H-30), 2.45 (m, 1H, H-19), 3.21 (m, 1H, H-3), 3.32 (d, *J* = 10.8 Hz, 1H, H-28), 3.81 (d, *J* = 10.8 Hz, 1H, H-28), 4.05 (m, 4H, 2xOCH₂CH₃) 5.42 (d, 1H, ²*J*_{PH} =18.6 Hz, H-29) -NMR (CDCl₃) δ (ppm): 14.7; 15.4; 15.9; 16.1; 16.4; 16.9; 18.3; 20.8; 25.8; 26.9; 27.3; 28.0; 29.2; 29.9; 34.1; 34.2; 37.1; 38.6; 38.9; 40.9; 42.7; 47.9; 49.7; 50.3; 51.5; 55.3; 60.4; 61.1; 61.2; 78.9; 111.0; 167.6.
   ³¹P-NMR (CDCl₃) δ (ppm): 18.54.
   IR (KBr, cm⁻¹) v: 3416 (OH), 1230 (P=O), 962 (P-O-R), 750 (P-C).
b) Z - 29 - Diethoxyphosphorylbetulin *(cis-2C)* (R = R₁ = Et)
   Yield: 13%. Mp. 126-128°C.
   TLC (methylene chloride/ethanol, 15:1, v/v); R_{f} = 0.28.
   Spectroscopic data of the obtained product are as follows:
   ¹H-NMR(CDCl₃) δ (ppm): 0.70 (m, 1H, H-5), 0.78 (s, 3H, CH3), 0.84 (s, 3H,CH₃), 0.987 (s, 3H, CH₃), 0.99 (s, 3H, CH₃),1.07 (s, 3H, CH₃), 1.33 (m, 6H, 2xOCH₂CH₃), 0.85-2.10 (m, 25H, CH, CH₂), 1.87 (br.s, 3H, H-30), 3.50 (m, 1H, H-19), 3.20 (m, 1H, H-3), 3.43 (d, *J* = 10.8 Hz, 1H, H-28), 3.81 (d, *J* = 10.8 Hz, 1H, H-28), 4.06 (m, 4H, 2xOCH₂CH₃) 5.28 (d, 1H, ²*J*_{PH} = 19.2 Hz, H-29).
   ¹³C-NMR (CDCl₃) δ (ppm): 14.7; 15.4; 15.9; 16.1; 16.4; 16.5; 18.3; 20.8; 21.4; 24.9; 27.0; 27.4; 28.0; 29.2; 29,3; 34.2; 34.3; 37.1; 37.2; 38.7; 38.9; 40.9; 42.7; 43.8; 47.9; 49.8; 50.5; 55.3; 60.5; 61.1; 61.2; 78.9; 111.9; 167.5.
   ³¹P-NMR (CDCl₃) δ (ppm): 17.91.
   IR (KBr, cm⁻¹) v: 3416 (OH), 1227 (P=O), 963 (P-O-R), 747 (P-C).

### Example 3. Preparation of phosphonate 2A (R = R₁ = Et)

Sodium hydroxide solution (0.47 M, 8 ml) in the water/tetrahydrofuran/methanol system (in the ratio of 1:2:1 v/v) and 0.133 g (0.2 mmol) of compound 4 (R = R₁ = Et) are placed in a round-bottomed flask, the whole mixture is stirred at room temperature for 5 hours. Then, the volatile components are evaporated on a vacuum evaporator. Methylene chloride (15 ml) is added to the residue and washed with 10% HCl and water. The organic layer is dried with anhydrous sodium sulfate (VI), then concentrated till dry. The product is purified by column chromatography (SiO₂, methylene chloride/ethanol, 15:1 v/v) yielding compound 2A (R = R₁ = Et).
a) 30 - Diethoxyphosphorylbetulin 2A (R = R₁ = Et)
   Yield: 68%. Mp. 160-162 °C.
   TLC (chloroform/ethanol, 15:1, v/v); R_{f}= 0.38.
   Spectroscopic data of the obtained product are as follows:
   ¹H-NMR (CDCl₃) δ (ppm): 0.70 (m, 1H, H-5), 0.78 (s, 3H, CH₃), 0.84 (s, 3H, CH₃), 0.99 (s, 3H, CH₃), 1.00 (s, 3H, CH₃), 1.04 (s, 3H, CH₃), 1.35 (m, 6H, 2x OCH₂CH₃), 0.80-2.20 (m, 25H, CH, CH₂), 2.42 (m, 1H, H-19), 2.60 (m, 2H, H-30), 3.21 (m, 1H, H-3), 3.34 (d, J= 10.8 Hz, 1H, H-28), 3.81 (d, *J* = 10.8 Hz, 1H, H-28), 4.13 (m, 4H, 2x OCH₂CH₃), 5.01 (br s., 1H, H-29), 5.05 (br s., 1H, H-29).
   ¹³C-NMR (CDCl₃) δ (ppm): 14.8; 15.4; 16.0; 16.1; 16.4; 16.5; 18.3; 20.8; 20.9; 26.5; 26.9; 27.0; 27.4; 28.0; 29.5; 30.7; 33.6; 34.2; 37.1; 38.7; 38.9; 40.9; 42.7; 47.8; 50.0; 50.3; 55.3; 61.5; 61.8; 61.9; 79.0; 112.7; 145.0.
   ³¹P-NMR (CDCl₃) δ (ppm): 27.81.
   IR (KBr, cm⁻¹) v: 3450 (OH), 1234 (P=O), 966 (P-O-R).

### Example 4. Preparation of acetylenic derivatives of 29-phosphonobetulin 5

(R = R₁ = Et, R₂ = HC≡CC(O)O, CH₃C≡CC(O)O, cyclo-PrC≡CC(O)O, R₃ = CH₂OC(O)C≡CH, CH₂OC(O)C≡CCH₃, CH₂OC(O)C≡C-Pr-cyclo)

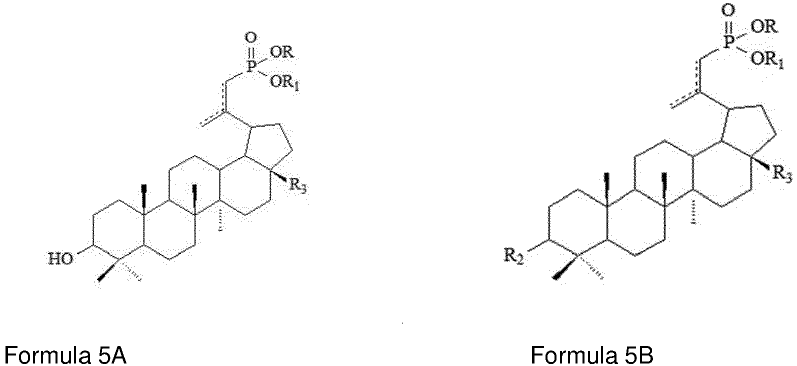

Compound 2B (R = R₁ = Et) in the amount of 0.29 g (0.5 mmol) is dissolved in 2.5 ml of methylene chloride. The obtained solution is cooled in an ice-water bath do -10°C, then 0.585 mmol of a corresponding acetylenecarboxylic acid (propiolic, 2-butynoic, cyclopropylpropiolic acid) is added, and next a solution of 0.123 g (0.595 mmol) of DCC (N,N'-dicyclohexylcarbodiimide) and 0.005g (0.04 mmol) of DMAP (4-dimethylaminopyridine) in 0.5 ml of methylene chloride is added dropwise. The reaction is carried out under argon atmosphere for 5 hours in a cooling bath, and then at room temperature. After 24 hours, the reaction mixture is filtered, the filtrate is concentrated till dry on a vacuum evaporator. The raw product is purified by column chromatography (SiO₂,methylene chloride/ethanol, 15:1, v/v), yielding diester 5B (R = R₁ = Et, R₂ = HC≡CC(O)O, CH₃C≡CC(O)O, cyclo-PrC≡CC(O)O, R₃ = CH₂OC(O)C≡CH, CH₂OC(O)C≡CCH₃, CH₂OC(O)C≡C-Pr-cyclo) and monoester 5A (R = R₁= Et, R₃ = CH₂OC(O)C≡CH, CH₂OC(O)C≡CCH₃, CH₂OC(O)C≡C-Pr-cyclo).
a) 29-Diethoxyphosphoryl-28-propynoylbetulin 5A (R = R₁ = Et, R₃= CH₂OC(O)C≡CH)
   Yield: 74%. Mp. 190-191°C.
   TLC (methylene chloride/ethanol, 15:1, v/v); R_{f} = O.52.
   Spectroscopic data of the obtained product are as follows:
   ¹H-NMR (CDCl₃) δ (ppm): 0.81 (m, 1H, H-5), 0.78 (s, 3H, CH₃), 0.84 (s, 3H, CH₃), 0.99 (s, 3H, CH₃), 1.04 (s, 3H, CH₃), 1.20 (s, 3H, CH₃) , 1.33 (m, 6H, 2x OCH₂CH₃), 1.06-2.01 (m, 24H, CH, CH₂), 2.07 (d, *J* = 3Hz, 3H, H-30), 2.47 (m, 1H, H-19), 2.93 (s, 1H, C=CH), 3.20 (m, 1H, H-3), 3.97 (d, *J* = 10.8 Hz, 1H, H-28), 4.05 (m, 4H, 2xOCH₂CH₃) 4.39 (d, *J* = 10.8 Hz, 1H, H-28), 5.43 (d, 1H, ²*J*_{PH} = 18 Hz, H-29).
   ¹³C-NMR (CDCl₃) δ (ppm): 14.7; 15.4; 16.0; 16.1; 16.4; 18.2; 20.7; 25.4; 25.8; 26.9; 27.3; 28.0; 29.6; 34.1; 34.6; 37.1; 37.5; 38.6; 38.9; 40.8; 42.7; 46.6; 49.7; 50.2; 51.4; 55.3; 61.1; 61.2; 64.6; 74.6; 74.9; 78.9; 111.9; 153.2; 166.8.
   31 P-NMR (CDCl₃) δ (ppm): 18.25
   IR (KBr, cm⁻¹) v: 3360 (O-H), 2107 (C≡C), 1713 (C=O), 1230 (P=O), 961 (P-O-R), 753 (P-C).
b) 29-Diethoxyphosphoryl-3,28-dipropynoylbetulin 5B (R = R₁ = Et, R₂ = HC≡CC(O)O, R₃ = CH₂OC(O)C≡CH)
   Yield: 10%. Mp. 129-133°C.
   TLC (methylene chloride/ethanol, 15:1, v/v) R_{f} = 0.74.
   Spectroscopic data of the obtained product are as follows:
   ¹H-NMR (CDCl₃) δ (ppm): 0.81 (m, 1H, H-5), 0.87 (s, 3H, CH₃), 0.90 (s, 3H, CH₃), 0.91 (s, 3H, CH₃), 0.99 (s, 3H, CH₃), 1.04 (s, 3H, CH₃), 1.33 (m, 6H, 2xOCH₂CH₃), 0.95-2.01 (m, 22H, CH, CH₂), 2.08 (d, *J* = 3Hz, 3H, H-30), 2.47 (m, 1H, H-19), 2.88 (br. s, 1H, C≡CH), 2.93 (br. s, 1H, C≡CH), 3.97 (d, *J* = 10.8 Hz, 1H, H-28), 4.05(m, 4H, 2xOCH₂CH ₃) 4.39 (d, *J* = 10.8 Hz, 1H, H-28), 4.62 (m, 1H, H-3), 5.43 (d, 1H, ²*J*_{PH} = 18.6 Hz, H-29).
   ¹³C-NMR (CDCl₃) δ (ppm): 14.7; 16.0; 16.1; 16.4; 18.1; 20.7; 23.5; 24.8; 25.5; 26.9; 27.9; 29.6; 33.7; 34.0; 34.6; 37.0; 37.4; 37.9; 38.3; 40.9; 42.7; 46.6; 49.4; 49.7; 50.1; 51.3; 55.3; 61.1; 61.2; 64.6; 74.1; 74.6; 74.9; 75.1; 83.6; 111.5; 152.8; 153.2; 166.7.
   ³¹P- NMR (CDCl₃) δ (ppm): 18.20.
   IR (KBr, cm⁻¹)v: 3326 (C≡C-H), 2115 (C≡C), 1715 (C=O), 1232 (P=O), 963 (P-OR), 754 (P-C).
c) 28-(2-butynoyl)-29-diethoxyphosphorylbetulin 5A (R = R₁ = Et, R₃ =CH₂OC(O)C≡CCH₃)
   Yield: 84%. Mp. 150-153°C.
   TLC (methylene chloride/ethanol, 15:1, v/v) R_{f} = 0.46.
   Spectroscopic data of the obtained product are as follows:
   ¹H-NMR (CDCl₃) δ (ppm): 0.69 (m, 1H, H-5), 0.77 (s, 3H, CH₃), 0.83 (s, 3H, CH₃), 0.98 (s, 6H, 2xCH₃), 1.03 (s, 3H, CH₃), 1.32 (m, 6H, 2xOCH₂CH₃), 0.8-2.10 (m, 24H, CH, CH₂), 2.01 (s, 3H, C≡CCH₃), 2.06 (d, *J* = 3Hz, 3H, H-30), 2.47 (m, 1H, H-19), 3.20 (m, 1H, H-3), 3.94 (d, *J* = 10.8 Hz, 1H, H-28), 4.04 (m, 4H, 2x OCH₂CH₃), 4,33 (d, J = H-28), 5,42 (d 1H, 2*J*_{PH} = 18 Hz, H-29)
   ¹³C-NMR (CDCl₃) δ (ppm): 3.9; 11.4; 14.7; 15.4; 16.0; 16.1; 16.4; 18.3; 20.7; 25.3; 25.8; 26.9; 27.3; 28.0; 29.1; 29.6; 34.1; 34.6; 37.1; 37.4; 38.6; 38.9; 40.9; 42.7; 46.5; 49.7; 50.2; 55.3; 61.1; 61.2; 64.0; 72.4; 78.9; 85.8; 111.0; 154.3; 167.0.
   ³¹P -NMR (CDCl₃) δ (ppm): 18.33.
   IR (KBr, cm⁻¹) v: 3414 (O-H), 2243 (C≡C), 1707 (C=O), 1247 (P=O), 1026 (P-O-C), 750 (P-C).
d) 3,28-Di(2-butynoyl)-29-diethoxyphosphorylbetulin 5B (R = R₁ = Et, R₂ = CH₃C≡CC(O)O, R₃ = CH₂OC(O)C≡CCH₃)
   Yield: 7%. Mp. 229-304°C.
   TLC (methylene chloride/ethanol, 15:1, v/v) R_{f} = 0.71.
   Spectroscopic data of the obtained product are as follows:
   1 H-NMR (CDCl₃) δ (ppm): 0.79 (m, 1H, H-5), 0.86 (s, 3H, CH₃), 0.89 (s, 6H, 2x CH₃), 0.97 (s, 3H, CH₃), 1.03 (s, 3H, CH₃), 1.32 (m, 6H, 2xOCH₂CH₃), 0.8-2.10 (m, 23H, CH, CH₂), 1.997 (s, 3H, C≡CCH₃), 2.01 (s, 3H, C≡CCH₃), 2.07 (d, *J* = 3Hz, 3H, H-30), 2.47 (m, 1H, H-19), 3.93 (d, *J* = 11.1 Hz, 1H, H-28), 4.04 (m, 4H, 2xOCH₂CH₃), 4.33 (d, *J* = 11.1 Hz, 1H, H-28), 4.59 (m, 1H, H-3), 5.42 (d, 1H, ²*J*_{PH} = 18 Hz, H-29).
   ¹³C-NMR (CDCl₃) δ (ppm): 3.9; 14.1; 14.7; 16.0; 16.1; 16.4; 16.5; 18.1; 20.7; 23.5; 25.5; 25.7; 26.9; 27.9; 29.6; 34.0; 34.6; 37.0; 37.4; 37.9; 38.3; 40.9; 42.7; 46.5; 49.7; 50.1; 50.2; 55.4; 61.1; 61.2; 64.0; 72.4; 72.8; 82.7; 85.0; 85.8; 111.4; 153.9; 154.3, 167.3.
   ³¹P-NMR (CDCl₃) δ (ppm): 18.30.
   IR (KBr, cm⁻¹) v:2241 (C≡C), 1710 (C=O), 1242 (P=O), 1059 (P-O-C), 748 (P-C)
e) 28-cyclopropylpropynoyl-29-diethoxyphosphorylbetulin 5A (R = R₁ = Et, R₃ =CH₂OC(O)C≡C-Pr-cyclo)
   Yield: 87%. Mp. 150-153°C.
   TLC (methylene chloride/ethanol, 15:1, v/v) R_{f} = 0.50.
   Spectroscopic data of the obtained product are as follows:
   ¹H-NMR (CDCl₃) δ (ppm): 0.69 (m, 1H, H-5), 0.70 (s, 3H, CH₃), 0.77 (s, 3H, CH₃), 0.98 (s, 6H, 2xCH₃), 1.02 (s, 3H, CH₃), 1.32 (m, 6H, 2x OCH₂CH₃), 0.8-2.10 (m, 29H, CH, CH₂), 2.06 (d, *J* = 2.7 Hz, 3H, H-30), 2.47 (m, 1H, H-19), 3.20 (m, 1H, H-3), 3.92 (d, *J* = 10.8 Hz, 1H, H-28), 4.04 (m, 4H, 2x OCH₂CH₃), 4.31 (d, *J* = 10.8 Hz, 1H, H-28), 5.42 (d, 1H, ²*J*_{PH} = 18 Hz, H-29).
   ¹³C-NMR (CDCl₃) δ (ppm): -0.1; 1.6; 9.8; 14.7; 15.3; 15.9; 16.5; 16.6; 16.9; 16.9;18.8; 21.3; 23.2; 26.3; 27.5; 27.9; 28.5; 30.2; 32.1; 34.7; 35.2; 37.7; 38.0; 39.2; 39.4; 41.4; 43.2; 47.1; 50.3; 50.8; 55.8; 61.7; 61.7; 64.4, 69.0; 79.5; 94.2; 112.5; 154.9; 167.9.
   ¹³P-NMR (CDCl₃) δ (ppm): 18.35.
   IR (KBr, cm⁻¹) v: 2243 (C≡C), 1707 (C=O), 1278 (P=O), 1038 (P-O-R), 750 (P-C).
f) 3,28-Di(cyclopropylpropynoyl)-29-diethoxyphosphorylbetulin 5B (R = R₁ = Et, R₂ = cyclo-PrC≡CC(O)O, R₃ = CH₂OC(O)C≡C-Pr-cyclo)
   Yield: 13%. Mp. 144-147°C.
   TLC (methylene chloride/ethanol, 15:1, v/v) R_{f} = 0.76.
   Spectroscopic data of the obtained product are as follows:
   ¹H-NMR (CDCl₃) δ (ppm): 0.79 (m, 1H, H-5), 0.85 (s, 3H, CH₃), 0.88 (s, 6H, 2x CH₃), 0.97 (s, 3H, CH₃), 1.02 (s, 3H, CH₃), 1.32 (m, 6H, 2x OCH₂CH₃), 0.8-2.10 (m, 33H, CH, CH₂), 2.06 (d, *J* = 3Hz, 3H, H-30), 2.46 (m, 1H, H-19), 3.91 (d, *J* = 11.1 Hz, 1H, H-28), 4.07 (m, 4H, 2x OCH₂CH₃), 4.31 (d, *J* = 10.8 Hz, 1H, H-28), 4.57 2 (m, 1H, H-3), 5.41 (d, 1H, ²*J*_{PH} = 18.6 Hz, H-29).
   ¹³C-NMR (CDCl₃) δ (ppm): -1.6; -0.1; 8.1; 8.2; 8.5; 13.7; 15.0; 15.1; 15.3; 15.4; 15.5; 17.1; 19.7; 22.5; 24.7; 25.8; 25.9; 26.9; 28.6; 29.5; 30.6; 33.0; 33.6; 36.0; 36.4; 36.9; 37.3; 39.8; 41.6; 45.5; 48.7; 49.1; 54.4; 60.1; 60.1; 62.8; 67.4; 67.9; 81.6; 91.7; 92.6; 111.9; 152.9; 153.4; 166.0.
e) 3,28-Di(cyclopropylpropynoyl)-29-diethoxyphosphorylbetulin 5B (R = R1 = Et, R2 = cyclo-PrC≡CC(O)O, R3 = CH2OC(O)C≡C-Pr-cyclo)
   Yield: 13%. Mp. 144-147°C.
   TLC (methylene chloride/ethanol, 15:1, v/v) Rt = 0.76.
   Spectroscopic data of the obtained product are as follows:
   ¹H-NMR (CDCl₃) δ (ppm): 0.79 (m, 1H, H-5), 0.85 (s, 3H, CH3), 0.88 (s, 6H, 2x CH3), 0.97 (s, 3H, CH3), 1.02 (s, 3H, CH3), 1.32 (m, 6H, 2x OCH2CH3), 0.8-2.10 (m, 33H, CH, CH2), 2.06 (d, J = 3Hz, 3H, H-30), 2.46 (m, 1H, H-19), 3.91 (d, J = 11.1 Hz, 1H, H-28), 4.07 (m, 4H, 2x OCH2CH3), 4.31 (d, J = 10.8 Hz, 1H, H-28), 4.57 (m, 1H, H-3), 5.41 (d, 1H, ²*J*_{PH} = 18.6 Hz, H-29).
   ¹³C-NMR (CDCl₃) δ (ppm): -1.6; -0.1; 8.1; 8.2; 8.5; 13.7; 15.0; 15.1; 15.3; 15.4; 15.5; 17.1; 19.7; 22.5; 24.7; 25.8; 25.9; 26.9; 28.6; 29.5; 30.6; 33.0; 33.6; 36.0; 36.4; 36.9; 37.3; 39.8; 41.6; 45.5; 48.7; 49.1; 54.4; 60.1; 60.1; 62.8; 67.4; 67.981.6; 91.7; 92.6; 111.9; 152.9; 153.4; 166.0.
   ³¹P-NMR (CDCl₃) δ (ppm): 18.33.
   IR (KBr, cm⁻¹) v: 2223 (C≡C), 1710 (C=O), 1265 (P=O), 1014 {P-O-R), 799 (P-C).

### Example 5. Preparation of 29-phosphonates of alkynyloxycarbonyl and alkyloxycarbonylbetulin 5

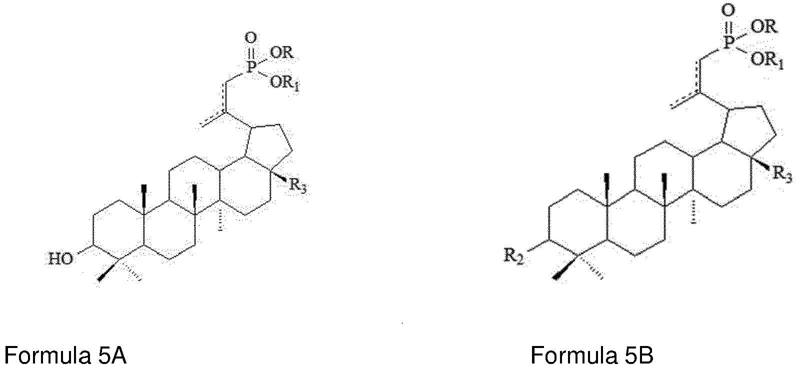

(R = R₁ = Et, R₂ = HC≡CCH₂OC(O)O, R₃ = CH₂OC(O)OCH₂C≡CH)

Compound 2B (R = R₁ = Et) (0.29 g, 0.5 mmol) is dissolved in 3 ml of benzene, 1.5 ml pyridine is added and cooled in an ice-salt bath to the temperature of about -5°C. Then, a solution of 1 mmol of the propargyl chloroformate w 2.5 ml of benzene is added dropwise and stirred for 18 hours at room temperature. After evaporation of volatile components, the residue is dissolved in 3 ml of chloroform and washed with 10% sulfuric (VI) acid and water. The extracts are dried with anhydrous sodium sulfate (VI) and concentrated under reduced pressure. The raw product is purified by column chromatography (SiO₂, chloroform/ethanol 15:1, v/v), yielding diester 5B (R = R₁ = Et, R₂ = HC≡CCH₂OC(O)O, R₃ =, CH₂OC(O)OCH₂C≡CH) and monoester 5A (R = R₁ - Et, R₃ = CH₂OC(O)OCH₂C≡CH).
a) 29-Diethoxyphosphoryl-28-propargyloxycarbonylbetulin 5A (R = R₁ = Et, R₃ = CH₂OC(O)OCH₂C≡CH)
   Yield: 63%. Mp. 140-142°C.
   TLC (methylene chloride/ethanol, 15:1, v/v) R_{f} = 0.49.
   Spectroscopic data of the obtained product are as follows:
   ¹H-NMR (CDCl₃) δ (ppm): 0.70 (m, 1H, H-5), 0.78 (s, 3H,CH₃), 0.84 (s, 3H, CH₃), 0.99 (s, 6H, 2x CH₃), 1.05 (s, 3H, CH₃), 1.33 (m, 6H, 2x OCH₂CH₃), 0.86-2.10 (m, 24H, CH, CH₂), 2.08 (br. s, 3H, H-30), 2.47 (m, 1H, H-19), 2.56 (t, *J* = 2.4 Hz, 1H, C≡CH), 3.21 (m, 1H, H-3), 3.95 (d, *J* = 10.8 Hz, 1H, H-28), 4.05 (m, 4H, 2xOCH₂CH₃) 4.38 (d, *J* = 10.8 Hz, 1H, H-28), 4.76 (m, 2H, OCH₂-C≡C), 5.43 (d, 1H, ²*J*_{PH} = 18 Hz, H-29).
   ¹³C-NMR (CDCl₃) δ (ppm): 13.7; 14.3; 14.9; 15.0; 15.1; 15.4; 17.2; 19.7; 24.8;25.9; 26.3; 27.0; 28.5; 28.6; 33.1; 33.5; 36.0; 36.1; 36.4; 37.6; 37.8; 39.8; 41.7; 45.7; 48.7; 49.2; 50.4; 54.2; 54.3; 60.1; 60;1; 66.0; 74.7; 77.9; 110.9; 154.0.
   ¹³P-NMR (CDCl₃) δ (ppm): 18.30.
   IR (KBr, cm⁻¹)v: 3437 (O-H), 2128 (C≡C), 1750 (C=O), 1250 (P=O), 969 (P-O-R), 789 (P-C).
b) 29-diethoxyphosphoryl-3,28-di(propargyloxycarbonyl)betulin 5B (R = R₁= Et, R₂ = HC≡CCH₂OC(O)O, R₃= CH₂OC(O)OCH₂C≡CH)
   Yield: 7%. Mp. 150-153°C.
   TLC (methylene chloride/ethanol, 15:1, v/v) R_{f} = 0.70.
   Spectroscopic data of the obtained product are as follows:
   ¹H-NMR (CDCl₃) δ (ppm): 0.80 (m, 1H, H-5), 0.87 (s, 3H, CH₃), 0.88 (s, 3H, CH₃), 0.94 (s, 3H, CH₃), 0.99 (s, 3H, CH₃), 1.05 (s, 3H, CH₃), 1.33 (m, 6H, 2x OCH₂CH₃), 0.82-2.10 (m, 23H, CH, CH₂), 2.08 (br. s, 3H, H-30), 2.48 (m, 1H, H-19), 2.54 (br. s, 1H, C≡CH), 2.56 (br. s, 1H, C≡CH), 3.95 (d, *J* = 10.8 Hz, 1H, H-28), 4.06 (m, 4H, 2x OCH₂CH₃), 4.38 (m, 2H, H-3, H-28), 4.74 (m, 2H, OCH₂-C≡C), 4.76 (m, 2H, OCH₂-C≡C), 5.43 (d, 1H, ²*J*_{PH} =18 Hz, H-29).
   ¹³C-NMR (CDCl₃) δ (ppm): 13.7; 14.9; 15.1; 15.3; 15.4; 17.0; 19.7; 22.5; 24.7; 25.9; 26.8; 28.5; 28.7; 33.0; 33.5; 36.0; 36.4; 37.0; 37.3; 39.8; 41.7; 45.7; 48.7; 49.1; 54.0; 54.3; 60.1; 66.0; 74.4; 74.7; 76.4; 79.2; 85.1; 110.9; 153.5; 154.0.
   31 P-NMR (CDCl₃) δ (ppm): 18.27.
   IR (KBr, cm⁻¹)v: 2131 (C≡C), 1757 (C=O), 1263 (P=O), 1026 (P-O-R), 800 (P-C).

The evaluation of cytotoxic activity of the compounds described with Formula 2 was carried out against human cancer cells [brain glioma SNB-19, breast cancer T47D, melanoma C32] using WST1 tests (Table 1).

**Table 1.**

| Compound symbol | Cytotoxic activity *in vitro* as IC₅₀ [µg/ml] | | |
|---|---|---|---|
| | T47D | SNB-19 | C32 |
| Betulin | 77.9±4 | 23.5±2.0 | |
| R = R₁ = Et, R₂ = AcO, R₃ = CH₂OAc | 58.0±3.4 | 51.9±0.8 | - |
| *trans* R = R₁ =Et, R₂= OH, R₃ = CH₂OH | 6.2±0.5 | 5.9±0.4 | - |
| *cis* R = R₁ = Et, R₂ =OH, R₃ = CH₂OH | 0.9±0.1 | 4.8±0.1 | - |
| R=R₁=Et, R₂=HC≡CC(O)O, R₃ = CH₂C(O)C≡CH | 2.8±0.6 | 0.6±0.1 | - |
| R = R₁ = Et, R₂ = HO, R₃ =CH₂C(O)C≡CH | 0.4±0.1 | 0.3±0.1 | - |
| R = R₁ =Et, R₂=CH₃C=CC(O)O, R₃ =CH₂OC(O)C≡CCH₃ | 13.9±3.4 | 7.6±1.0 | 4.9±0.3 |
| R = R₁ =Et, R₂ =HO, R₃ =CH₂OC(O)C ≡ CCH₃ | 9.6±0.6 | 7.5±0.3 | 4.9±0.1 |
| R = R₁ = Et, R₂ = HC≡CCH₂UC(U)O, R₃ = CH₂OC(O)OCH₂C≡CH | Neg | 0.5±0.1 | 51.7±4.8 |
| R = R₁ = Et, R₂ = HO, R₃ = CH₂OC(O)OCH₂C≡CH | 49.4±6.1 | 24.5±12.6 | 5.9±0.3 |
| R =R₁ =Et, R₂ =cyclo-PrC≡CC(O)O, R₃ =CH₂OC(O)C≡C-cycloPr | 79.5±1.9 | 40.4±6.8 | 9.4±0.2 |
| R = R₁ = Et, R₂ =HO, R₃ = CH₂OC(O)C≡C-cycloPr | 8.2±0.6 | 2.2±0.8 | 4.7±0.7 |
| cisplatin | 55.9±4.5 | 5.3±0.1 | 4.3±0.6 |

| | | | |
|---|---|---|---|
| Neg - no activity in the applied concentration range - no assay | | | |

Phosphonates of acetylenic betulin derivatives obtained according to the invention may be used preferably for inhibition of cancer cells proliferation.

## Claims

1. Phosphonates of acetylenic betulin derivatives with Formula 2, 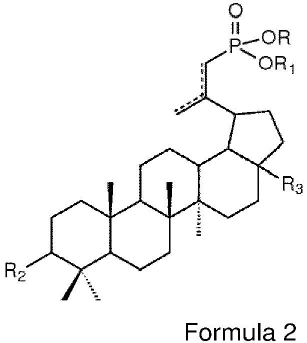 wherein a phosphonic group is bounded with C29 or C30 carbon atom of the isopropenyl group, the dashed lines between C20, and C29 and C30 indicate that the bond between them may be a double or single bond, and the individual substituents are as follows:
R is alkyl group (C1-C4),
R₁ is alkyl group (C1-C4),
R₂ is hydroxy, propynoyloxy, cyclopropylpropynoyloxy, 2-butynoyloxy, propargyloxycarbonyloxy group,
R₃ is hydroxymethyl, propynoyloxymethyl, cyclopropylpropynoyloxymethyl, 2-butynoyloxymethyl, propargyloxycarbonyloxymethyl group,

2. A method for preparation of phosphonates of acetylenic betulin derivatives with Formula 2 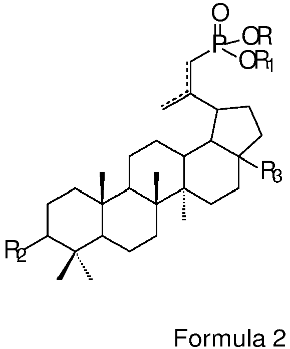 wherein R, R₁, R₂ and R₃ are defined in claim 1, comprising the steps of:
(a) reaction a compound, having a structure of Formula 3 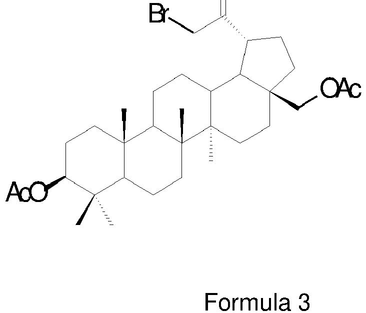 with a trialkyl phosphite at a temperature of 100°C-165°C, giving a product with Formula 4 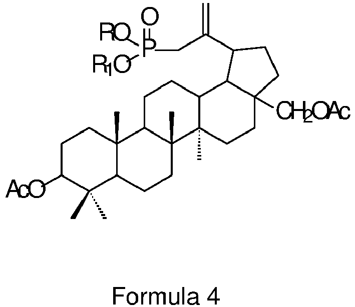
(b) hydrolysis of the product of step (a) in alkaline medium in the presence of KOH in ethanolic solution, at boiling temperature, giving a mixture of allyl-vinyl rearrangement products with Formula 2B (trans) and 2C (cis), wherein R = R₁ is alkyl group (C1 - C4), 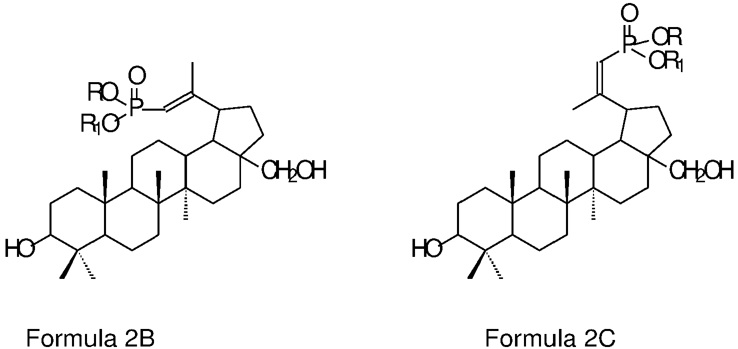 or in the presence of NaOH in CH₃OH/THF/H₂O solution at room temperature, giving a not-rearranged compound with Formula 2A, wherein R = R₁ is alkyl group (C1 - C4),

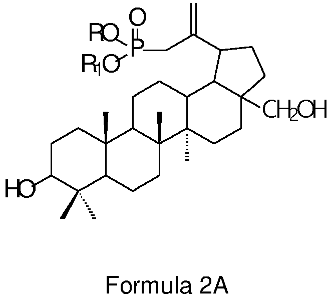

3. Phosphonates of acetylenic betulin derivatives having a structure defined in claim 1 for use in inhibition of cancer cells proliferation.

## Patentansprüche

1. Phosphonate der acetylenischen Betulin-Derivate gemäß Formel 2, 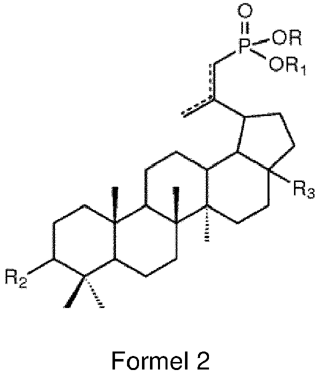 worin eine Phosphongruppe mit dem Kohlenstoffatom C29 oder C30 der Isopropenylgruppe in Verbindung steht, die gestrichelte Linien zwischen C20 sowie C29 und C30 zeigen, dass die Bindungsstärke zwischen diesen eine Doppel- oder Einzelbindung sein kann, an, und worin die einzelnen Substituenten die Folgenden sind:
R ist eine Alkylgruppe (C1-C4),
R₁ ist eine Alkylgruppe (C1-C4),
R₂ ist eine Hydroxyl-, Propinoyloxyl-, Cyclopropylpropinoyloxyl-, 2-Butynoiloxyl-, Propargyloxycarbonyloxyl-Gruppe,
R₃ ist eine Hydroxymethyl-, Propinoyloxymethyl-, Cyclopropylpropinoyloxymethyl-, 2-Butynoiloxymethyl-, Propargyloxycarbonyloxymethyl-Gruppe

2. Verfahren zur Herstellung von Phosphonaten der acetylenischen Betulin-Derivate gemäß Formel 2 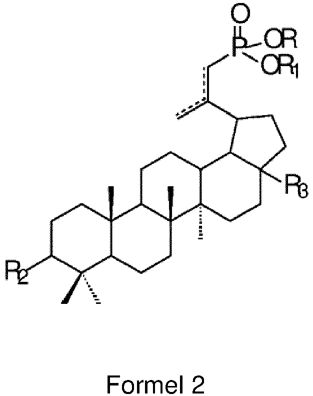 worin R, R₁, R₂, R₃ unter Anspruch 1 definiert sind, aus den folgenden Schritten bestehend:
(a) Reaktion einer Verbindung, mit der Struktur gemäß Formel 3 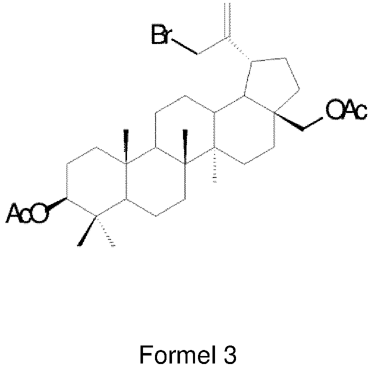 mit einem Trialkylphosphit bei einer Temperatur von 100-165 °C, wobei ein Produkt gemäß Formel 4 entsteht 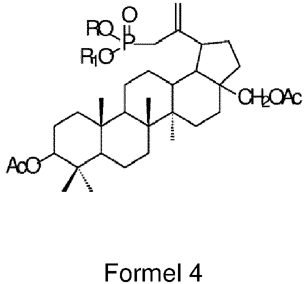
(b) Hydrolyse des Produkts aus Schritt (a) in einem alkalischen Medium in der Gegenwart von KOH in einer Ethanollösung bei Siedetemperatur, wobei eine Mischung aus Allyl-Vinyl-Neuordnungsprodukten gemäß Formel 2B (trans) sowie 2C (cis) entsteht, wobei R = R₁ eine Alkylgruppe (C1-C4) ist, 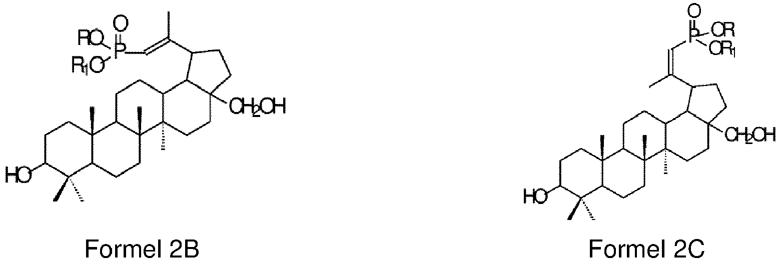 oder in der Gegenwart von NaOH in einer CH₃OH/ THF/ H₂O-Lösung bei Raumtemperatur, wobei ein nicht neu geordnetes Produkt gemäß Formel 2A entsteht, wobei R = R₁ eine Alkylgruppe (C1-C4) ist,

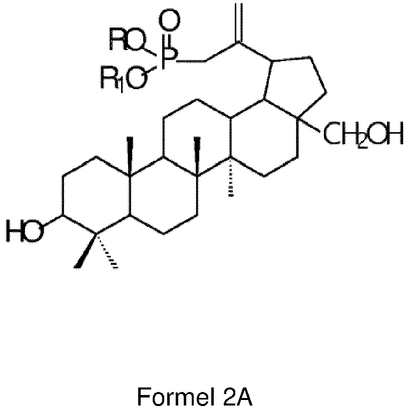

3. Phosphonate der acetylenischen Betulin-Derivate mit der unter Anspruch 1 beschriebenen Struktur, zum Einsatz in der Unterdrückung der Ausbreitung von Krebszellen.

## Revendications

1. Phosphonates de dérivés acétyléniques de bétuline de formule 2, 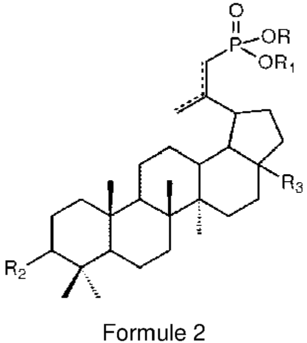 où un groupe phosphonique est lié à un atome de carbone C29 ou C30 du groupe isopropényle, les lignes pointillées entre C20, C29 et C30 indiquent que la liaison entre eux peut être une liaison double ou simple, et les substituants individuels sont les suivants :
R est un groupe alkyle (C1-C4),
R₁ est un groupe alkyle (C1-C4),
R₂ est un groupe hydroxy, propynoyloxy, cyclopropylpropynoyloxy, 2-butynoyloxy, propargyloxycarbonyloxy,
R₃ est un groupe hydroxyméthyle, propynoyloxyméthyle, cyclopropylpropynoyloxyméthyle, 2-butynoyloxyméthyle, propargyloxycarbonyloxyméthyle,

2. Procédé de préparation de phosphonates de dérivés acétyléniques de bétuline de formule 2 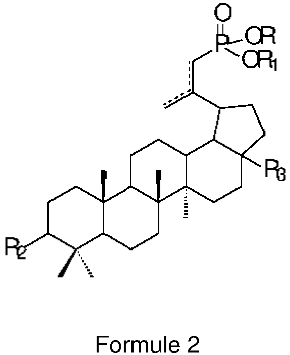 où R, R₁, R₂ et R₃ sont définis dans la revendication 1, comprenant les étapes de :
(a) réaction d'un composé ayant une structure de la formule 3 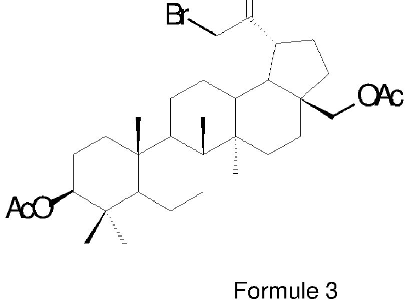 avec un phosphite de trialkyle à une température de 100°C à 165°C, donnant un produit de formule 4 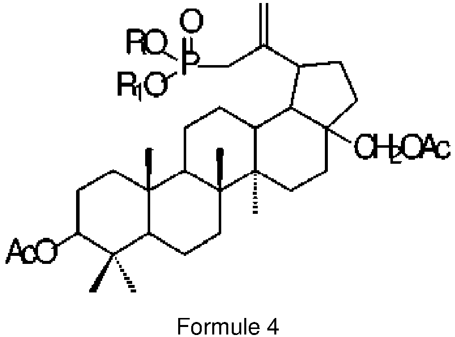
(b) hydrolyse du produit de l'étape (a) en milieu alcalin en présence de KOH en solution éthanolique, à température d'ébullition, donnant un mélange de produits de réarrangement allyl-vinyle de formule 2B (trans) et 2C (cis), dans lequel R = R₁ est un groupe alkyle (C1-C4), 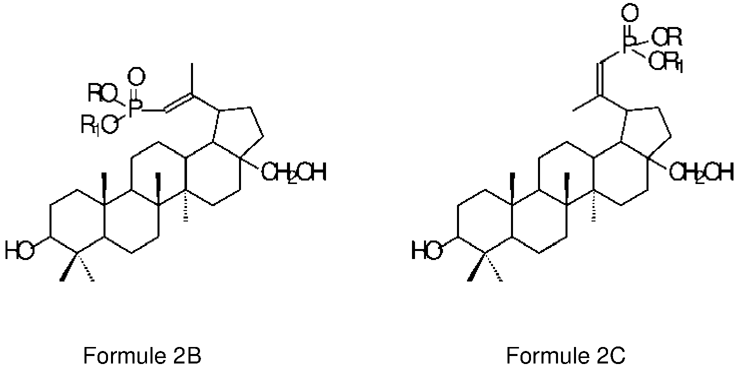 ou en présence de NaOH dans une solution CH₃OH/THF/H₂O à température ambiante, donnant un composé non réarrangé de formule 2A, dans lequel R = R₁ est un groupe alkyle (C1-C4),

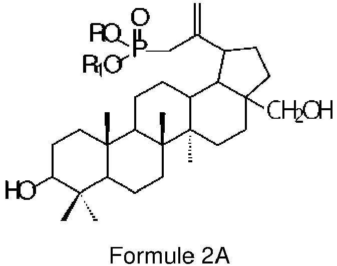

3. Phosphonates de dérivés acétyléniques de bétuline ayant une structure définie dans la revendication 1 pour une utilisation dans l'inhibition de la prolifération des cellules cancéreuses.
